(19) Europäisches Patentamt
European Patent Office
Office européen des brevets

(11) Publication number : **0 623 616 A1**

# (12) EUROPEAN PATENT APPLICATION

(21) Application number : **94400962.0**

(22) Date of filing : **03.05.94**

(51) Int. Cl.$^5$ : **C07D 413/12,** A61K 31/445, A61K 31/42

(30) Priority : **04.05.93 HU 9301284**

(43) Date of publication of application : **09.11.94 Bulletin 94/45**

(84) Designated Contracting States : **AT BE CH DE DK ES FR GB GR IT LI NL SE**

(71) Applicant : **EGIS GYOGYSZERGYAR RT.**
**Kereszturi ut 30-38**
**H-1106 Budapest (HU)**

(72) Inventor : **Gacsályi, István**
**Tàrogotò ùt 64/b**
**HU-1021 Budapest (HU)**
Inventor : **Budai, Zoltán**
**3 Lukács u.**
**HU-1023 Budapest (HU)**
Inventor : **Reiter, Klára**
**32/b Tövis u.**
**HU-1022 Budapest (HU)**
Inventor : **Mezei, Tibor**
**4 Borz u.**
**HU-1221 Budapest (HU)**
Inventor : **Blasko, Gábor**
**21 Molnár E. u.**
**HU-1113 Budapest (HU)**
Inventor : **Simig, Gyula**
**25 Hollosy S. u.**
**HU-1126 Budapest (HU)**
Inventor : **Fekete, Márton**
**49 Fo u.**
**HU-1027 Budapest (HU)**
Inventor : **Szemerédi, Katalin**
**3 Vörösvári u.**
**HU-1035 Budapest (HU)**
Inventor : **Egyed, András**
**58 Ujvidék u.**
**HU-1145 Budapest (HU)**
Inventor : **Gyertyán, István**
**33 Koronafürt u.**
**HU-1165 Budapest (HU)**
Inventor : **Schmidt, Eva**
**64/b Tárogato u.**
**HU-1021 Budapest (HU)**

(74) Representative : **Hirsch, Marc-Roger et al**
**Cabinet Hirsch**
**34 rue de Bassano**
**F-75008 Paris (FR)**

(54) **Heterocyclic compounds and process for the preparation thereof.**

(57) The invention relates to new heterocyclic compounds, a process for the preparation thereof, pharmaceutical compositions comprising the same, to the use of the said heterocyclic compounds for the treatment of diseases and for the preparation of pharmaceutical compositions suitable for the treatment of diseases.

According to the invention the new heterocyclic compounds the general formula (I), wherein A stands for a group of formula -C≡C- or -C=C-,

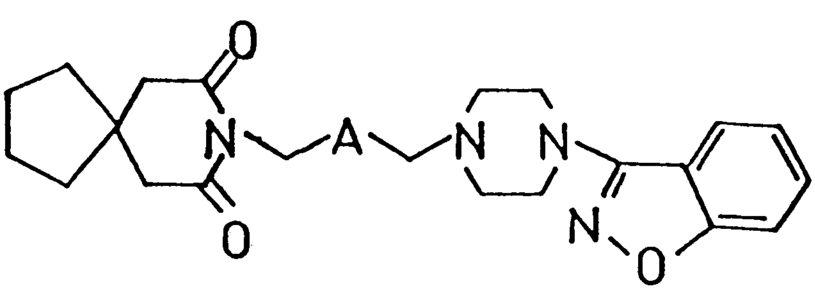

(I)

The new compounds according to the invention possess anxio-selective properties and can be used with advantage in the therapy.

EP 0 623 616 A1

This invention relates to new heterocyclic compounds, a process for the preparation thereof, pharmaceutical compositions comprising the same, to the use of the said heterocyclic compounds for the treatment of diseases and for the preparation of pharmaceutical compositions suitable for the treatment of diseases.

According to an aspect of the present invention there are provided new heterocyclic compounds of general formula (I),

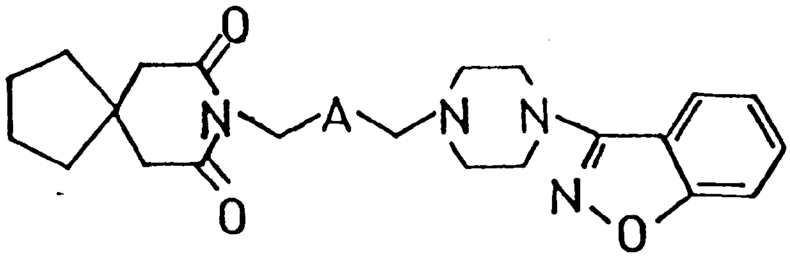

(I)

wherein A stands for a group of formula -C≡C- or -CH=CH-, and pharmaceutically acceptable acid-addition salts thereof.

The European patent specification No.196,096 describes imide derivatives having antipsychotic activity, which are structurally similar to the compounds according to the invention. The latter compounds, however, exhibit a fully different effect, they have anxioselective properties devoid of any neuroleptic character.

The Japanese Kokai Tokkyo Koho patent specification No.63-10,760 provides bicycloheptanes exhibiting tranquillant activity.

The Japanese Kokai Tokkyo Koho patent specification No.63-10,760 describes N-(piperazinylbutyl)-imide derivatives exerting tranquillant and antipsychotic effects.

The Hungarian patent specifications Nos 190,997 and 195,806 relate to benzothiazole and benzoxazole derivatives having antipsychotic activity. A preferred representative of the disclosed compounds is the thiaspiron of formula (II)

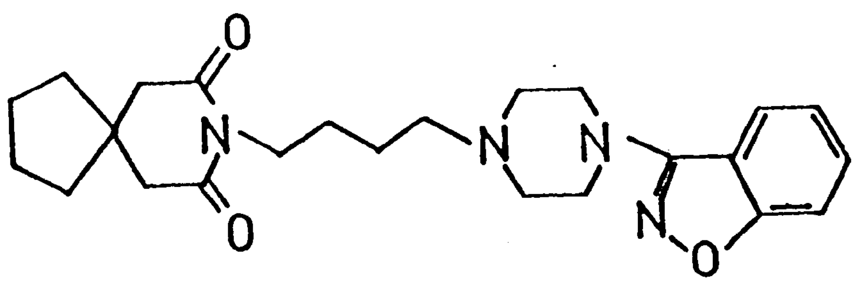

(II)

It is surprising that although the compounds according to the invention are structurally similar to the prior art compounds, they exert fully different activities.

According to another aspect of the present invention there is provided a process for the preparation of new compounds of general formula (I) and acid-addition salts thereof, which comprises

a) for the preparation of a compound of general formula (I), wherein A represents a group of formula -C≡C-, subjecting 8-azaspiro[4,5]decane-7,9-dione-8-propyne(2) with 1-(1,2-benzisothiazol-3-yl)piperazine to Mannich condensation; or

b) for the preparation of a compound of general formula (I), wherein A stands for a group of formula -C≡C-, reacting 8-azaspiro[4,5]decane-7,9-dione-8-propyne(2) with an alkyl magnesium halide of formula R-Mg-Hal, wherein R stands for alkyl having 1 to 4 carbon atom(s) and Hal denotes chlorine, bromine or iodine, reacting the thus-obtained propyne magnesium halide of general formula (III),

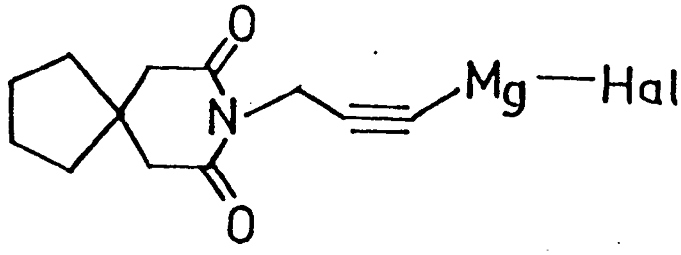

(III)

wherein Hal is as stated above, with at least one molar equivalent of trioxymethylene or formaldehyde, converting the butynol derivative of formula (IV)

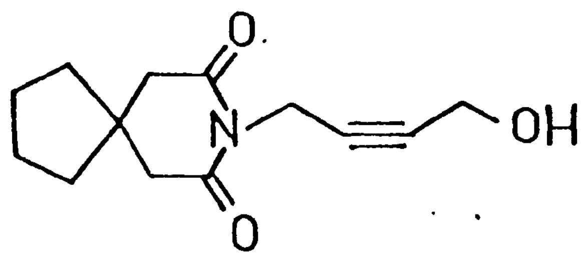

(IV)

thus obtained into a reactive derivative of general formula (V),

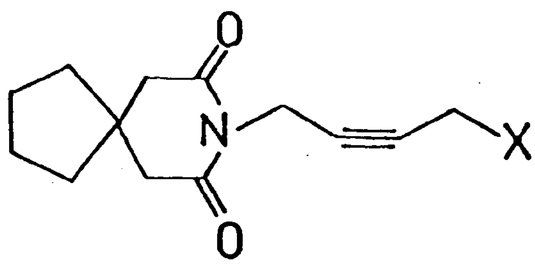

(V)

wherein X stands for a reactive ester group, and reacting the compound of general formula (V) with 1-(1,2-benzisothiazol-3-yl) piperazine; or

c) for the preparation of a compound of general formula (I), wherein A stands for a group of formula -CH=CH-, subjecting the compound of general formula (I) containing a group of formula -C≡C- in place of A to partial reduction;

and, if desired, converting a compound of general formula (I) to an acid-addition salt thereof or liberating a compound of general formula (I) from an acid-addition salt thereof.

The 8-azaspiro[4,5]decane-7,9-dione-8-propyne(2) used as starting substance in the process according to the invention is prepared according to the method specified in the Hungarian patent specification No.197,313.

According to variant a) of the process of the invention a compound of general formula (I) containing a group of formula -C≡C- in the place of A is prepared by subjecting 8-azaspiro[4,5]decane-7,9-dione-8-propyne(2) with 1-(1,2-benzisothiazol-3-yl)-piperazine to Mannich condensation. The Mannich condensation is carried out by methods known per se [Colvin, A. Buchler, Donald, E. Pearson: Survey of Organic Syntheses (USA, 1970) Vol. 1, page 465]. For the reaction formaldehyde is ensured in the form of paraformaldehyde or an aqueous formaline solution. The reaction is carried out under heating, preferably at a temperature between 30°C and the boiling point of the solvent.

The reaction is carried out in a inert solvent. For this purpose an organic solvent or water can be used. As organic solvent preferably ethers (e.g. diethyl ether, dioxane or tetrahydrofuran) or alcohols (e.g. methanol, ethanol, isopropanol etc.) can be used. Water can preferably be applied as solvent, when formaldehyde is used in the form of an aqueous solution. The desired compound of general formula (I) can be obtained by methods known per se (e.g. by extraction carried out with an appropriate organic solvent).

In the first step of variant b) of the process according to the invention 8-azaspiro[4,5]decane-7,9-dione-8-propyne(2) is reacted with an alkyl magnesium halide of general formula R-Mg-Hal, wherein R stands for a straight-chained or branched alkyl having 1 to 4 carbon atom(s) (e.g. methyl, ethyl, propyl etc.). For the preparation of the compound of formula R-Mg-Hal preferably methyl iodide, methyl bromide, methyl chloride, ethyl iodide, ethyl bromide or ethyl chloride is applied as starting substance. The reduction of the propyne(2) derivative with the compound of general formula R-Mg-Hal can be carried out in ether medium, under heating.

The compound of general formula (III) thus obtained is reacted - preferably without isolation - with at least one molar equivalent of trioxymethylene or formaldehyde. Preferably gaseous formaldehyde is applied. The trioxymethylene or formaldehyde can be used in an amount of 1 to 1.1, preferably 1.0 to 1.05, molar equivalent. The reaction is preferably carried out under heating. The thus-obtained compound of formula (IV) is separated from the reaction mixture by evaporating the ether solution.

The alcohol of formula (IV) is converted into a reactive ester of formula (V), wherein X preferably stands for halogen (e.g. chlorine, bromine or iodine), alkylsulfonyloxy (e.g. methylsulfonyloxy) or arylsulfonyloxy (e.g. phenylsulfonyloxy, p-bromophenylsulfonyloxy or p-tosyloxy). According to a preferred embodiment of the process of the invention the compound of formula (IV) is reacted with p-toluenesulfonyl chloride. The reaction can be carried out at room temperature or under mild heating.

The compound of formula (V) thus obtained is then reacted - after isolation or preferably in situ - with 1-(1,2-benzisothiazol-3-yl)-piperazine by methods known per se. The reaction is preferably carried out in an inert organic solvent. As reaction medium preferably aromatic hydrocarbons(e.g. benzene, toluene or xylene) can be used.

The reaction is preferably carried out under heating. It is particularly preferable to perform the reaction at the boiling point of the reaction mixture. The thus-obtained product can be isolated e.g. by the evaporation of the solution.

The compound of formula (VI),

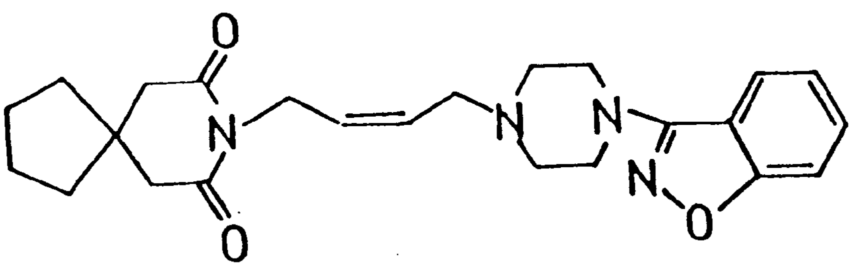

(VI)

namely 8-{4-[4-(1,2-benzisothiazol-3-yl)-1-piperazinyl]-but-2-enyl}-8-aza-spiro[4,5]-decane-7,9-dione, also exhibits valuable pharmaceutical properties.

According to variant c) of the process according to the invention the compound of formula (VI) is prepared by subjecting 8-{4-[4-(1,2-benzisothiazol-3-yl)-1-piperazinyl]-but-2-ynyl}-8-azaspiro[4,5]decane-7,9-dione of formula (VII)

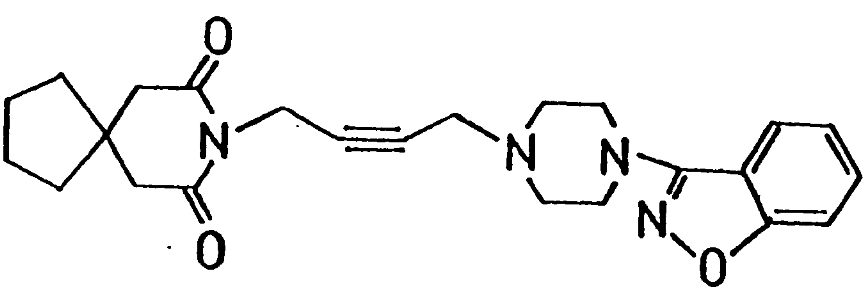

(VII)

to reduction. The reduction is carried out by catalytic hydrogenation, by methods known per se under circumstances suitable for the reduction of the triple bond into a double bond. As catalyst preferably palladium is used, optionally applied onto a carrier (e.g. coal). The reaction is carried out at a temperature about room temperature, under atmospheric pressure. The reduction can be carried out in a inert solvent. As reaction medium e.g. ethers (such as diethyl ether, tetrahydrofuran or dioxane) or alcohols (such as methanol or ethanol) can be used.

The compound of general formula (I) thus-obtained is converted into an acid-addition salt by methods know per se. For the salt formation preferably pharmaceutically acceptable inorganic or organic acids (such as hydrogen chloride, hydrogen bromide, sulfuric, nitric, maleic, fumaric, lactic, malic, succinic, tartaric acid etc.) can be applied. The salt formation is preferably carried out by reacting the base of general formula (I) in an inert organic solvent with a molar equivalent amount of the appropriate acid.

The compound of general formula (I) can be liberated from an acid-addition salt thereof with an appropriate base, by methods known per se.

The compounds of general formula (I) possess valuable pharmaceutical properties. They exert anxioselective activity devoid of any neuroleptic effect. The activity of the compounds of general formula (I) is shown by the following tests using as reference compounds some drugs available in the commerce.

1. Anxiolytic activity

1.1 Vogel test (drinking conflict test)

Male Wistar rats of 160 to 180 g body weight were kept free of food and drinking water for 24 and 48 hours, respectively. Test and carrier substances were administered intraperitoneally half an hour before testing. Animals within the experimental chamber were provided with drinking water through an inserted tube. After the animals' each twenty lapping for water the device emitted a 2 mA intensity electric shock through the drinking tube. During 5 minutes the shocks tolerated by the animals in order to quench their thirst were counted. The effect of treatment was expressed as the % increase of the tolerated shocks. The minimum effective dose (MED) was determined for each test compound [Vogel, J.R., Beer, B., Clody, D.E.:Psychopharmacologia (Berl.)21, 1(1971)]. The obtained data are summarized below.

| Compound (N° of Example) | MED (mg/kg) |
|---|---|
| 1 | 5.0 |
| Meprobamate | 25.0 |
| Chlorodiazepoxide | 10.0 |

The compound of formula (VII) according to Example 1 is 5 times as effective as Meprobamate and twice as effective as chlorodiazepoxide.

1.2 Elevated plus maze test on rat

The test was carried out with the aid of a wooden plus-shaped maze elevated to a height of 50 cm. Two arms (100 cm long and 15 cm wide) - opposite to one another - were enclosed up to a height of 40 cm along their longer sides and at the end. The other two arms were without walls (open arms). The central 15x15 cm part was open. Male rats belonging to the Spraghe Dawley strain and weighing 220 to 260 g were used as test animals. After pretreatment lasting 60 minutes the animals were placed into the central part of the maze. During the 5 minutes observation period the following parameters were recorded:

- time spent on the open arms
- time spent on the closed arms
- number of open arm entries
- number of closed arm entries.

The drug effect was expressed as percent increase of the time spent on the open arms and number of open arm entries. The minimum effective dose which caused a significant increase of the time spent on the open arms was calculated for every substance [Pelow, S., Chopin, P., File, S.E., Briley, M.:J. Neurosci. Methods 14, 149-167 (1985)]. The data are showed below.

| Compound (No. of Example) | MED (mg/kg), p.o. |
|---|---|
| 1 | 2,5 |
| Ritanserin | 5,0 |
| Meprobamate | >10,0 |

The compound according to Example 1 proved to be twice as effective as Ritanserin.

2. Sedative effect

2.1 Effect on the spontaneous motor activity

The test was carried out according to the method of Borsy et al. Groups consisting of 3 mice each were treated orally with different doses of the compounds to be tested 1 hour after treatment with the test compound or the vehicle the animals were placed into a 10-channel Dews system equipment. In this equipment the number of interruptions of infrared beam within 30 minutes was counted. From these date 50% inhibiting doses ($IC_{50}$) were determined with the aid of a line of regression [Borsy, J., Csanyi, E., Lazar, I.: Arch. Int. Pharmacodyn. 124, a (1960)]. The data obtained are shown below.

| Compound (No. of Example) | Mobility inhibition $ID_{50}$(mg/kg) |
|---|---|
| 1 | ineffective(>100) |
| Chlorodiazepoxide | 56,0 |
| Diazepam | 23,0 |
| Thiaspiron* | 8,4 |

* M.S. Eison, J.L.Minielli, J.S. Mew, D.D. Taylor: Drugs of Future 10(1I), 731 (1985).

The compound of Example 1 - contrary to diazepam and chlorodiazepoxide - does not influence the spontaneous motor activity of mice, consequently it is devoid of the unfavourable sedative side effect.

3. Antipsychotic effect

3.1 Conditioned avoidance response inhibition

The antipsychotic (neuroleptic) effect was measured by the inhibition of the learned conditioned avoidance reflex. The male Wistar rats used for the study were of 120 to 150 g body weight at the beginning of learning. A shuttle box was used as experimental device, it consisted of two parts, 24x24,5x23 cm each, separated by a wall with a 6x9 cm gate. In response to a suitable warning stimulus, in order to avoid the punishing (unconditioned) stimulus, animals within the box passed through the gate from one part to the other. The warning, i.e. conditioned stimulus (CS), a white light (1 Hz) blinking for 15 seconds, was applied at the actual place of the animal. The unconditioned stimulus (US), in form of 0,6 mA intensity electric shock, was randomly applied to the paw during the last 5 seconds of the conditioned stimulus. The animal's movement during the CS and US from one part of the box to the other was defined as avoidance and escape response, respectively. Both responses ceased the actual stimulus and stopped the trial. The time elapsed until the next trial (intertrial interval, ITI) was 15 seconds. While one experiment was carried out daily, an experiment consisted of 80 trials. Learning efficiency was expresses as percentage of the successful to the total avoidances. Effect of the neuroleptic drugs was examined in animals with stabilized conditioned reflexes and with at least 75% learning efficiency. Dosing was carried out once a week, one hour before the measurement in the shuttle box. To calculate the neuroleptic effect (50% inhibiting dose, $ID_{50}$), the results obtained after the treatment were compared to those obtained on the previous day (controls). The thus-obtained data are shown below.

| Compound (No. of Example) | Conditioned relfex $ID_{50}$ (mg/kg) |
|---|---|
| 1 | 30,0 |
| Chloropromazine | 13,2 |
| Haloperidol | 0,6 |
| Thiaspiron* | 10,5 |

* J.P. Yevich, J.S. Mew, D.W. Smith, W.G. Lobeck, J.D. Catt, J.L. Minielli, M.S. Eison, D.P. Taylor, L.A. Riblet, D.L. Temple Jr., J. Med. Chem. 29(3), 363(1986); M.S. Eison, J.L. Minielli, J.S. Mew, D.D. Taylor: Drugs of Future 10(II), 731(1985).

From the above results it can be established that the compound of Example 1 - contrary to the classical neuroleptics - does not influence the conditioned avoidance response up to an oral dose of 30 mg/kg. This dose range is much higher than the anxiolytic one.

According to the results of the experiments the anxio-selective effect of the compound of Example 1 is surprisingly new in the given structural range. The anxioselective effect is very favourable to ease human anxiety reactions, as such a compound probably does not influence the normal activity and does not cause somnolence or weariness. As anxiolytic, the compound can preferably be used for the treatment of generalized anxiety, post-traumatic stress reactions and for influencing social phobias.

According to a further aspect of the present invention there are provided pharmaceutical compositions comprising as active ingredient a compound of general formula (I) or a pharmaceutically acceptable acid-addition salt thereof in admixture with suitable inert solid or liquid pharmaceutical carriers. The pharmaceutical compositions of the present invention can be prepared by methods known per se by admixing the active ingredient with suitable inert solid or liquid carriers and bringing the mixture to galenic form.

The pharmaceutical compositions of the present invention may be suitable for oral (e.g.tablet, pill, coated pill, dragée, solid or soft gelatin capsule, solution, emulsion or suspension), parenteral (e.g. injection solution) or rectal (e.g. suppository) administration.

As carrier for the preparation of tablets, coated tablets, dragées and solid gelatin capsules e.g. lactose, corn starch, potato starch, talc, magnesium carbonate, magnesium stearate, calcium carbonate, stearic acid or the salts thereof can be used. As carrier for the soft gelatin capsules e.g. vegetable oils, fats, waxes or polyols of suitable consistency can be used. As carriers for the solutions and syrups e.g. water, polyols (polyethylene glycol), saccharose or glucose can be used. The injection solutions can comprise e.g. water, alcohols, polyols, glycerol or vegetable oils as carrier. The suppositories can be prepared with the aid of e.g. oils, waxes, fats or polyols of suitable consistency.

In addition, the pharmaceutical formulations may comprise auxiliaries usually applied in the pharmaceutical industry, e.g. wetting, sweetening agents, aroma substances, salts causing the change of osmotic pressure, buffers etc. The pharmaceutical formulations may further comprise other active ingredients, too.

It is preferred to finish the compounds of general formula (I) to dosage forms having an active ingredient content of 0.5 to 1000 mg, preferable 2.5 to 100 mg.

According to a further aspect of the present invention there is provided the use of the compounds of general formula (I) or pharmaceutically acceptable acid-addition salts thereof for the preparation of pharmaceutical compositions having particularly anxioselective properties.

According to a still further aspect of the present invention there is provided a method of anxioselective treatment, which comprises administering to a patient an effective amount of a compound of general formula (I) or a pharmaceutically acceptable acid-addition salt thereof.

The invention also relates to the compounds of formula (I) for use as an active therapeutic substance.

Also, the inventin relates to the compounds of formula (I) for use as an active therapeutic substance having anxio-selective properties.

Further details of the present invention are to be found in the following Examples without limiting the scope of protection to the said Examples (the melting points are uncorrected).

Example 1

8-{4-[4-(1,2-Benzisothiazol-3-yl)-1-piperazinyl]-but-2-ynyl}-8-azaspiro[4,5]decane-7,9-dione

Method A)

Into a 250 ml round-bottomed flask equipped with a stirrer and reflux condenser 205 g (0,1 mole) of 8-aza-spiro[4,5]decane-7,9-dione-8-propyne(2), 25 cm3 of dioxane, 23.0 g (0,105 mole) of 1-(1,2-benzisothiazol-3-yl)-piperazine, 3,6 g of paraformaldehyde and 0,2 of copper (II) acetate are measured. The reaction mixture is boiled for 3 hours, then cooled to room temperature, poured into water and extracted three times with 50 ml each of benzene. The benzene solutions are combined, treated with 0,5 g of activated carbon, and the solvent is removed on a hot water bath. The residue is diluted with n-hexane, the crystals are filtered off and washed with a mixture of cyclohexane and n-hexane. Thus 37,9 g of the desired compound are obtained.

Yield: 87% M.p.: 118.9-199.6°C (ethanol)

Analysis for the formula $C_{24}H_{28}N_4O_2S$ (436,56):

| | | | | |
|---|---|---|---|---|
| Calculated: | C%=66.03 | H%=6.64 | N%=12.83 | S%=7.35 |
| Found: | C%=65.86 | H%=6.54 | N%=12.78 | S%=7,26 |

Method B)

Into a round-bottomed flask equipped with a stirrer, reflux condenser, thermometer and dropping funnel 20.5 g (0.1 mole) of 8-azaspiro[4,5]decane-7,9-dione-8-propyne-(2), 67,5 g(62.5 cm3, 0.81 mole) of aqueous formaldehyde solution and 0,2 g of copper(II) acetate are measured, then 21.9 g (0.1 mole) of 1-(1,2-benzisothiazol-3-yl)piperazine are added to the mixture under stirring. The heterogeneous reaction mixture is heated slowly to a temperature of 80 to 82°C and stirred for further one hour. The completion of the reaction is controlled by thin-layer chromatography (Kieselgel $60F_{254}$, benzene-methanol 4:1). The reaction mixture is then cooled to room temperature and the product is extracted three times with 50 cm3 each of dichloroethane. The organic phase is dried by stirring with 5.0 g of anhydrous potassium carbonate for 15 minutes. The solvent is distilled off under reduced pressure, the thus-obtained product is suspended in n-hexane and the crystals are filtered off and washed with 1:1 mixture of n-hexane and cyclohexane. Thus 39.7 g of the desired product are obtained.

Yield:91% M.p.:118-119.0°C (ethanol)

The quality of the product is equal to that of the substance obtained according to method A).

Salt formation:

The base prepared according to variant A) or B) is dissolved in hot ethanol, and an equivalent amount of fumaric acid is added to it. The hydrogen fumarate salt crystallizes upon cooling.

M.p.:164-167°C (ethanol)

| Analysis for the formula $C_{28}H_{32}N_4O_6S$ (552.64): | | | | |
|---|---|---|---|---|
| Calculated: | C%=60.85 | H%=5.84 | N%=10.14 | S%=5.80 |
| Found: | C%=60.63 | H%=5.93 | N%=10.00 | S%=5.72 |

Example 2

Tablet comprising 25 mg of active ingredient.

Composition of one tablet is as follows:

| | |
|---|---|
| . Active ingredient | 15.0 mg |
| . Corn starch | 97.0 mg |
| . Polyvinylpyrrolidone | 175.0 mg |
| . Magnesium stearate | 3.0 mg |
| | 300.0 mg |

This tablet is prepared as follows:

The active ingredient and the corn starch are admixed, then wetted with 10-15% by weight of aqueous polyvinylpyrrolidone solution. The mixture is granulated and then dried at a temperature of 40 to 50°C. The dry granules are rubbed through a sieve, mixed with talcum and magnesium stearate and tablets are prepared from the mixture.

The weight of one tablet is 300 mg.

Example 3

| | |
|---|---|
| . Active ingredient | 250.0 mg |
| . Lactose | 270.0 mg |
| . Corn starch | 75.0 mg |
| . Magnesium stearate | 5.0 mg |
| | 600.0 mg |

The tablet is prepared as follows:

The active ingredient, the lactose and the corn starch - are wetted and mixed, granulated and dried at a temperature of 40 to 50°C. The dry granules are rubbed through a sieve as described hereinabove, mixed with magnesium stearate and- tablets are formed.

The weight of one tablet is 600.0 mg.

Example 4

Dragée comprising 25.0 mg of active ingredient

The composition of one dragée core is as follows:

| | |
|---|---|
| . Active ingredient | 25.0 mg |
| . Lactose | 245.0 mg |
| . Corn starch | 18.0 mg |
| . Gelatin | 8.0 mg |
| . Magnesium stearate | 4.0 mg |
| | 300.0 mg |

The active ingredient and the corn starch are mixed and wetted with 10% by weight aqueous gelatin solution. Granules are formed from the wet mixture and the granules are dried at a temperature of 40 to 50°C. The dry granules are rubbed through a sieve, homogenized with talcum and magnesium stearate and dragee cores of 300.0 mg are compressed from the mixture.

Example 5

Dragée comprising 50.0 mg of active ingredient

The composition of one dragee core is as follows:

| | |
|---|---|
| . Active ingredient | 50.0 mg |
| . Lactose | 97.0 mg |
| . Polyvinylpyrrolidone | 2.0 mg |
| . Magnesium stearate | 1.0 mg |
| | 150.0 mg |

The granules are prepared as described hereinabove. The weight of the dragée cores is 150.0 mg.

The dragée cores are coated with a layer containing sugar and talcum in a manner known per se. The dragée thus obtained is painted with non-toxic food paint to the desired colour and polished with bee-wax.

Example 6

Gelatin capsule comprising 5.0 mg of active ingredient
The composition of one gelatine capsule is as follows:

| | |
|---|---|
| . Active ingredient | 5.0 mg |
| . Corn starch | 40.0 mg |
| . Aerosil | 3.0 mg |
| . Magnesium stearate | 2.0 mg |
| | 50.0 mg |

The components are homogenized and filled into gelatin capsules of suitable size.

Example 7

Gelatin capsule comprising 25.0 mg of active ingredient
The composition of one gelatine capsule is as follows:

| | |
|---|---|
| . Active ingredient | 25.0 mg |
| . Corn starch | 265.0 mg |
| . Aerosil | 6.0 mg |
| . Magnesium stearate | 4.0 mg |
| | 300.0 mg |

The ingredients are homogenized and filled into gelatin capsules of suitable size.

Example 8

Gelatin capsule comprising 250.0 mg of active ingredient
The composition of one gelatine capsule is as follows:

| | |
|---|---|
| . Active ingredient | 250.0 mg |
| . Lactose | 148.0 mg |
| . Magnesium stearate | 2.0 mg |
| | 400.0 mg |

The ingredients are homogenized and filled into gelatin capsules of suitable size.

Example 9

Injection comprising 25.0 mg of active ingredient
The composition of one ampoule is follows:
. Active ingredient 25.0 mg
. Sodium chloride 5.0 mg
dissolved in 5 cm3 of twice-distilled water.

The active ingredient and the sodium chloride are dissolved in the necessary amount of twice-distilled water suitable for making injections. The solution is filtered, filled into ampoules and sterilized.

Example 10

Injection comprising 50.0 mg of active ingredient
The composition of one ampoule is follows:
. Active ingredient 50.0 mg
. Sodium chloride 10.0 mg

The active ingredient and the sodium chloride are dissolved in the necessary amount of twice-distilled water, then filled into ampoules under sterile conditions.

Example 11

Suppository comprising 250.0 mg of active ingredient
The composition of one suppository is as follows:

| | |
|---|---|
| . Active ingredient | 250.0 mg |
| . Fatty acid glyceride | 750.0 mg |
| | 1000.0 mg |

The fatty acid glyceride is melted and the active ingredient is homogenized, then poured into a mould. One suppository weights 1000.0 mg and comprises 250.0 mg of active ingredient.

Example 12

Drop comprising 5% of active ingredient
The composition of one drop is as follows:

| | |
|---|---|
| . Active ingredient | 50.0 mg |
| . Sorbitol | 340.0 mg |
| . Polyethylene glycol | 100.0 mg |
| . Citric acid | 1.0 mg |
| . Sodium citrate | 3.0 mg |
| . Ion-free water | 1.0 mg |
| . Flavourant | 1.0 mg |
| | 505.0 mg |

The sorbitol, the active ingredient, the citric acid and the sodium citrate are dissolved in the aqueous solution of propylene glycol, then after dissolution of the solid materials the flavourant is added. The solution is filtered and filled into flasks supplied with a drop-dispenser.

## Claims

**1.-** Compounds of general formula (I),

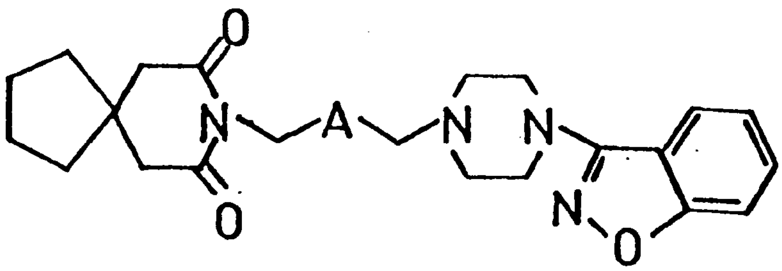

(I)

wherein A stands for a group of formula -C≡C- or -CH=CH-, and pharmaceutically acceptable acid-addition salts thereof.

**2.-** A process for the preparation of compounds of general formula (I), wherein A stands for a group of formula -C≡C- or -CH=CH-, and pharmaceutically acceptable acid-addition salts thereof, which comprises

a) for the preparation of a compound of general formula (I), wherein A represents a group of formula -C≡C-, subjecting 8-azaspiro[4,5]decane-7,9-dione-8-propyne(2) with 1-(1,2-benzisothiazol-3-yl)piperazine to Mannich condensation; or

b) for the preparation of a compound of general formula (I), wherein A stands for a group of formula -C≡C-, reacting 8-azaspiro[4,5]decane-7,9-dione-8-propyne(2) with an alkyl magnesium halide of formula R-Mg-Hal, wherein R stands for alkyl having 1 to 4 carbon atom(s) and Hal denotes chlorine, bromine or iodine, reacting the thus-obtained propyne magnesium halide of general formula (III),

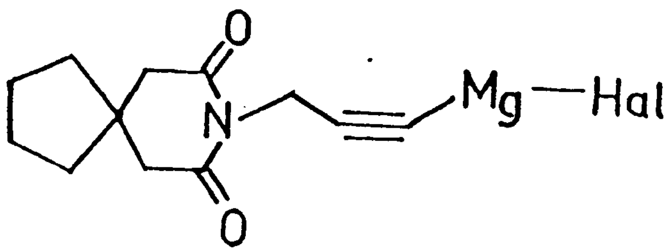

(III)

wherein Hal is a stated above, with at least one molar equivalent of trioxymethylene or formaldehyde, converting the butynol derivative of formula (IV)

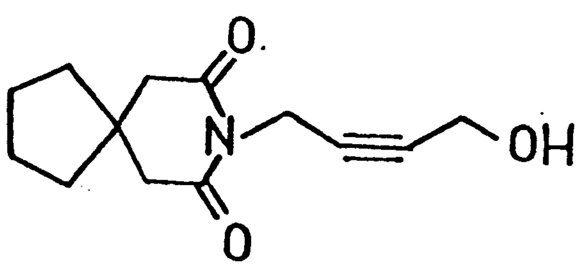

(IV)

thus obtained into a reactive derivative of general formula (V),

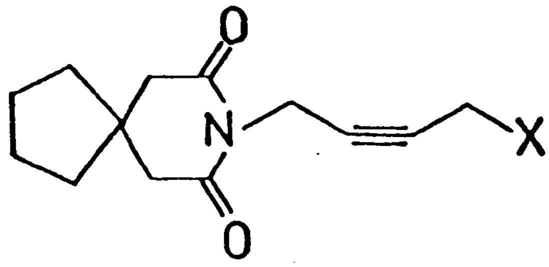

(V)

wherein X stands for a reactive ester group, and reacting the compound of general formula (V) with 1-(1,2-benzisothiazol-3-yl) piperazine; or

c) for the preparation of a compound of general formula (I), wherein A stands for a group of formula -CH=CH-, subjecting the compound of general formula (I) containing a group of formula -C≡C- in place of A to partial reduction;

and, if desired, converting a compound of general formula (I) to an acid-addition salt thereof or liberating a compound of general formula (I) from an acid-addition salt thereof.

**3.-** A process as claimed according to variant a) of claim 2, which comprises carrying out the Mannich condensation by using paraformaldehyde.

**4.-** A process as claimed according to variant a) of claim 2, which comprises carrying out the Mannich condensation by using an aqueous formaldehyde solution.

**5.-** A process according to variant b) of claim 2, which comprises using for the preparation of the compound of general formula R-Mg-Hal methyl iodide, methyl chloride, ethyl bromide or ethyl chloride.

**6.-** A process according to variant b) of claim 2, which comprises carrying out the reaction of the compound of general formula (III) with trioxymethylene or gaseous formaldehyde under heating.

**7.-** A process according to variant c) of claim 2, which comprises carrying out the reaction in the presence of palladium catalyst.

**8.-** Pharmaceutical compositions comprising as active ingredient a compound of general formula (I) or a pharmaceutically acceptable acid-addition salt thereof according to claim 1, in admixture with suitable inert solid or liquid pharmaceutical carriers.

**9.-** Compounds of general formula (I), or pharmaceutically acceptable acid-addition salts thereof according to claim 1, for use as an active therapeutic substance.

**10.-** Compounds of general formula (I) or pharmaceutically acceptable acid-addition salts thereof, according to claim 1, for use as an active therapeutic substance having anxioselective properties.

**11.-** Use of compounds of general formula (I) or pharmaceutically acceptable acid-addition salts thereof for the preparation of medicament having particularly anxioselective properties.

European Patent Office

# EUROPEAN SEARCH REPORT

Application Number
EP 94 40 0962

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| D,A | EP-A-0 196 096 (SUMITOMO PHARMACEUTICALS COMPANY LTD.) 1 October 1986<br>* the whole document *<br>--- | 1-11 | C07D413/12<br>A61K31/445<br>A61K31/42 |
| D,Y | J. MED. CHEM.<br>vol. 29 , 1986<br>pages 359 - 369<br>J.P.YEVICH ET AL 'Synthesis and biological evaluation of 1-(1,2-benzisothiazol-3-yl)- and (1,2-benzisoxazol-3-yl)piperazine derivatives as potential antipsychotic agents'<br>*see especially compounds 24 and 30*<br>--- | 1-11 | |
| Y | GB-A-2 114 119 (BRISTOL-MEYERS COMPANY) 17 August 1983<br>* the whole document *<br>--- | 1-11 | |
| D,Y | PATENT ABSTRACTS OF JAPAN<br>vol. 12, no. 213 (C-505)17 June 1988<br>& JP-A-63 010 760 (SUMITOMO PHARMACEUTICAL CO. LTD.) 18 January 1988<br>* abstract *<br>--- | 1-11 | TECHNICAL FIELDS SEARCHED (Int.Cl.5)<br>C07D<br>A61K |
| Y | PATENT ABSTRACTS OF JAPAN<br>vol. 12, no. 213 (C-505)17 June 1988<br>& JP-A-63 010 786 (SUMITOMO PHARMACEUTICAL CO LTD.) 18 January 1988<br>* abstract *<br>----- | 1-11 | |

The present search report has been drawn up for all claims

| Place of search | Date of completion of the search | Examiner |
|---|---|---|
| MUNICH | 9 August 1994 | Scruton-Evans, I |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)